# EUROPEAN PATENT SPECIFICATION

(11) **EP 4 262 760 B1**
(45) Date of publication and mention of the grant of the patent: **24.09.2025**
(21) Application number: 21839539.0
(22) Date of filing: 17.12.2021
(51) Int. Cl.: A61K 9/20, A61K 9/28, A61K 31/444

(54) **EDOXABAN FORMULATION CONTAINING NO SUGAR ALCOHOLS**
EDOXABAN FORMULIERUNG OHNE ZUCKERALKOHOLE
FORMULATION D'ÉDOXABAN SANS ALCOOLS DE SUCRE

(30) Priority: 18.12.2020 EP 20215680
(43) Date of publication of application: 25.10.2023
(73) Proprietor: KRKA, d.d., Novo mesto, 8000 Novo mesto (SI)
(72) Inventor: BARIC, Matej, 8000 Novo mesto (SI); SVETIC, Sandi, 8000 Novo mesto (SI); KORASA, Klemen, 8000 Novo mesto (SI); HOTKO, Anka, 8000 Novo mesto (SI); CELIC, Tadeja Birsa, 8000 Novo mesto (SI)
(74) Representative: Kutzenberger Wolff & Partner
(86) International application number: PCT/EP2021/086507
(87) International publication number: WO 2022/129535

(56) References cited:
- EP-A1- 3 744 320
- WO-A1-2013/026553
- WO-A1-2016/020080

## Description

The invention relates to a tablet comprising Edoxaban and to a process for the manufacture of the tablet. The tablet comprises a core and optionally a coating encapsulating said core, wherein the core comprises Edoxaban, a pharmaceutically acceptable salt and/or solvate thereof; and one or more saccharides comprising or essentially consisting of a mixture of glucose and maltose, optionally together with one or more oligosaccharides, being dextrates; wherein the core does not comprise sugar alcohol.

Edoxaban is an anticoagulant medication and a direct factor Xa inhibitor useful i.a. for preventing or treating deep vein thrombosis, pulmonary embolism, or blood clots in patients with nonvalvular atrial fibrillation.

Coated tablets containing Edoxaban are marketed under the tradename Lixiana^{®}. According to the assessment report of the European Medicine Agency (2015), the product is presented as immediate release tablets containing equivalent doses amounting to 15 mg, 30 mg or 60 mg of Edoxaban in form of the tosilate salt. Other ingredients in the tablet core are mannitol, pregelatinized starch, crospovidone, hydroxypropylcellulose, and magnesium stearate. The core is coated with a filmcoat containing hypromellose (E464), macrogol 8000, titanium dioxide (E171), talc, carnauba wax, iron oxide yellow (E172), iron oxide red (E172). The manufacturing process consists of four main steps: fluid bed granulation; blending; tableting; filmcoating. The tablets are manufactured from a common granulate and are quantitatively proportional.

EP 2 140 867 discloses pharmaceutical compositions containing Edoxaban, a pharmacologically acceptable salt thereof, or a hydrate of any of these, and one or more species selected from the group consisting of a sugar alcohol and a water-swelling additive.

EP 2 444 087 relates to a solid pharmaceutical composition containing Edoxaban or a pharmacologically acceptable salt thereof, or a solvate thereof, wherein the content of Edoxaban is 0.5% by weight or more and less than 15% by weight with respect to the total weight of the pharmaceutical composition.

EP 2 548 556 discloses a method for producing a pharmaceutical composition containing Edoxaban, comprising the step of mixing Edoxaban or a pharmaceutically acceptable salt thereof, or a solvate thereof, one or more excipients selected from the group consisting of a sugar alcohol and a water-swelling additive, a disintegrant, and a binder under conditions for keeping the maximum water content of the granules during granulation at 10% or less.

EP 2 548 879 relates to a crystal form of Edoxaban p-toluenesulfonate monohydrate, and a method for producing the same.

EP 2 742 941 relates to a pharmaceutical composition containing Edoxaban or a pharmacologically acceptable salt thereof, or a solvate thereof, and an acid or a salt thereof.

EP 3 177 290 discloses a pharmaceutical composition comprising Edoxaban, or a pharmaceutically acceptable salt thereof, a water soluble vinylpyrrolidone polymer selected from the group consisting of povidone and copovidone, and a cellulose ether, and not comprising a sugar alcohol.

EP 3 549 585 relates to an orally disintegrating tablet comprising Edoxaban, a pharmacologically acceptable salt thereof, or a solvate thereof, an organic acid, 0.1 to 2.0% by weight of a water-soluble polymer, and a disintegrant.

WO 2013/026553 relates to compositions and dosage forms containing Edoxaban and a CO₂-forming agent, preferably for immediate release.

WO 2020/022824 discloses a formulation comprising Edoxaban as an active ingredient and pea starch as coating agent.

WO 2021/123192 relates to solid pharmaceutical formulations comprising edoxaban as an active ingredient, namely tablets comprising edoxaban, a first diluent and a second diluent in its composition, and to a process for producing the same.

EP 3 744 320 discloses the combination of lactose as a water-soluble filler and crospovidone and sodium starch glycolate as disintegrants.

CN 103919746 relates to an edoxaban sustained release tablet consisting of the following components in percentage by weight: 14-21 percent of edoxaban p-toluene sulfonate hydrate, 0-36 percent of hydroxypropyl methylcellulose, 0-28 percent of carbomer, 10-28 percent of lactose, 29-38 percent of diluent, 0-3 percent of povidone and 0.6-4 percent of lubricant.

CN 108743556 discloses an edoxaban tablet, which is prepared from edoxaban tosilate or a monohydrate thereof, a surfactant, a pH value adjuster, a dissolution enhancer, a filler, a disintegrator, a coating agent, a lubricant and water, wherein the edoxaban tosilate or the monohydrate thereof, the surfactant, the pH value adjuster and the dissolution enhancer are dissolved in an aqueous solution,and granulation is performed to obtain the granulated product.

The Edoxaban formulations of the prior art are not satisfactory in every respect and there is a demand for improved Edoxaban formulations. The prior art pharmaceutical formulations for oral administration comprising Edoxaban have a similar dissolution rate and bioavailability as the registered product Lixiana^{®} but it would be desirable to still improve these formulations with regard to several aspects.

Despite positive effects of sugar alcohols, their consumption is frequently linked to irritable bowel syndrome (IBS) and abnormal flatulence, which affect quality of life negatively and result in a considerable economic burden in terms of health-care costs. Consumption of pentitol- and hexitol-type polyols and disaccharide polyols may cause gastrointestinal disturbances at least in unaccustomed subjects. The occurrence of disturbances depends on consumer properties and on the molecular size and configuration of the polyol molecule (KK Mäkinen, Int J Dent. 2016: 5967907).

It is an object of the invention to provide Edoxaban formulations having advantages to the Edoxaban formulations of the prior art and processes for the manufacture of such formulations. The formulations should preferably be obtainable by easy processing and in a reproducible manner, while having good flowability and superior content uniformity of Edoxaban. Due to the intolerance of many people to sugar alcohols, such as sorbitol, the use of sugar alcohols should preferably be avoided. Additionally, due to the intolerance of many people to lactose, the use of lactose should preferably be avoided. Further, the formulation should preferably provide a superior and pH independent dissolution rate and bioavailability of Edoxaban, as well as at the same time preferably have superior storage stability.

This object has been achieved by the subject-matter of the patent claims.

It has been surprisingly found that tablets can be prepared which comprise Edoxaban, a pharmaceutically acceptable salt and/or solvate thereof, which do not contain sugar alcohol, which do not require water soluble vinylpyrrolidones, and which provide satisfactory dissolution profiles. Further, it has been surprisingly found that mechanically stable tablets comprising Edoxaban, a pharmaceutically acceptable salt and/or solvate thereof, which do not contain lactose, can be prepared using dextrates, i.e. a mixture of glucose and maltose.

A first aspect of the invention relates to a tablet comprising a core and optionally a coating encapsulating said core, wherein the core comprises or essentially consists of
- Edoxaban, a pharmaceutically acceptable salt and/or solvate thereof;
- one or more saccharides comprising or essentially consisting of a mixture of glucose and maltose, optionally together with one or more oligosaccharides, being dextrates;
- optionally, one or more binders;
- optionally, one or more disintegrants; and
- optionally, one or more lubricants;
wherein the core does not comprise sugar alcohol.

Unless expressly stated otherwise, all percentages are weight percent. Unless expressly stated otherwise, all percentages are related to the total weight of the tablet according to the invention. When the tablet according to the invention is coated, the total weight encompasses the weight of the core and the coating.

Unless expressly stated otherwise, *"essentially consist of"* and *"essentially consisting of"* means that the presence of other material or substances is at most 5 wt.-%, preferably at most 2.5 wt.-%, more preferably at most 1.0 wt.-%, most preferably at most 0.1 wt.-%. Preferably, *"essentially consist of"* means *"consist of"* whereas *"essentially consisting of"* means *"consisting of",* respectively.

The core of the tablet according to the invention may contain optional components, namely independently of one another one or more binders, one or more disintegrants, and/or one or more lubricants. Preferably, the core of the tablet contains all said components. The core of the tablet according to the invention may contain additional pharmaceutical excipients that are known to the skilled person such as carriers, fillers, diluents, compaction agents, antioxidants, buffering agents, chelating agents, dispersion enhancers, surfactants, colorants, sweeteners, and the like.

Suitable excipients are known to the skilled person and are described e.g. in P.J. Sheskey et al., Handbook of Pharmaceutical Excipients, Pharmaceutical Press, 8th ed. 2017; SC Smolinske, CRC Handbook of food, drug, and cosmetic excipients, CRC Press, 1992.

The tablet according to the invention comprises a core and optionally a coating encapsulating said core. When the tablet according to the invention comprises no coating, the tablet according to the invention preferably essentially consists of the core. When the tablet according to the invention comprises a coating, the tablet according to the invention preferably essentially consists of the core and the coating.

The core of the tablet according to the invention comprises one or more saccharides selected from glucose, fructose, disaccharides, oligosaccharides, namely saccharides comprising or essentially consisting of a mixture of glucose and maltose, optionally together with one or more oligosaccharides, being dextrates.

For the purpose of the specification, disaccharides encompass 2 sugar units (independently of one another in furanose form or pyranose form) linked to one another through a glycosidic bond. Preferred glycosidic bonds are selected from alpha-1,4, beta-1,4, alpha-1,6 and beta-1,6.

For the purpose of the specification, oligosaccharides encompass at least 3 sugar units (independently of one another in furanose form or pyranose form) linked to one another through glycosidic bonds. Preferred glycosidic bonds are independently of one another selected from alpha-1,4, beta-1,4, alpha-1,6 and beta-1,6. Preferably, oligosaccharides encompass not more than 10 sugar units; for the purpose of the specification, such saccharides encompassing more than 10 sugar units are referred to as polysaccharides.

Preferably, the one or more saccharides according to the invention encompass no sugar units other than glucose and maltose.

Unless expressly stated otherwise, glucose, fructose and galactose as well as the respective units thereof independently of one another are present as D-enantiomers, i.e. D-glucose, D-glucose unit, D-fructose, D-fructose unit, D-galactose, and D-galactose unit, respectively.

The core of the tablet according to the invention does not comprise sugar alcohol. When the core is coated, the coating preferably does not contain sugar alcohol either. Preferably, the tablet according to the invention does not contain sugar alcohol.

Sugar alcohols are known to the skilled person. Typical representatives include but are not limited to mannitol, erythritol, xylitol, sorbitol, maltitol, inositol and lactitol.

In preferred embodiments, the one or more saccharides comprise one or more oligosaccharides. Preferably, the one or more oligosaccharides comprise or consist of semi-digested starch, maltodextrin, pullulan, β-cyclodextrin, or mixtures thereof.

Preferably, the weight content of the one or more saccharides, which is a mixture of glucose and maltose, optionally together with one or more oligosaccharides, is at least 10 wt.-%; preferably at least 15 wt.-%, more preferably at least 20 wt.-%, still more preferably at least 25 wt.-%, yet more preferably at least 30 wt.-%, even more preferably at least 35 wt.-%, most preferably at least 40 wt.-%, and in particular at least 45 wt.-%; in each case relative to the total weight of the one or more saccharides and relative to the total weight of the tablet.

Preferably, the weight content of the one or more saccharides, which is a mixture of glucose and maltose, optionally together with one or more oligosaccharides, is at most 90 wt.-%; preferably at most 85 wt.-%, more preferably at most 80 wt.-%, still more preferably at most 75 wt.-%, yet more preferably at most 70 wt.-%, even more preferably at most 65 wt.-%, most preferably at most 60 wt.-%, and in particular at most 55 wt.-%; in each case relative to the total weight of the one or more saccharides and relative to the total weight of the tablet.

Preferably the weight content of the one or more saccharides, which is a mixture of glucose and maltose, optionally together with one or more oligosaccharides, is within the range of from 0.1 to 80 wt.-%, preferably 0.1 to 70 wt.-%, more preferably 0.1 to 60 wt.-%, still more preferably 0.1 to 50 wt.-%, yet more preferably 0.1 to 40 wt.-%, most preferably 0.1 to 30 wt.-%; in each case relative to the total weight of the one or more saccharides and relative to the total weight of the tablet.

Preferably, the weight content of the one or more saccharides, which is a mixture of glucose and maltose, optionally together with one or more oligosaccharides, is within the range of from 1.0 to 60 wt.-%, preferably 1.0 to 50 wt.-%; in each case relative to the total weight of the one or more saccharides and relative to the total weight of the tablet.

Preferably, the weight content of the one or more saccharides, which is a mixture of glucose and maltose, optionally together with one or more oligosaccharides, is within the range of 48±16 wt.-%; preferably 48±14 wt.-%, more preferably 48±12 wt.-%, still more preferably 48±10 wt.-%, yet more preferably 48±8.0 wt.-%, even more preferably 48±6.0 wt.-%, most preferably 48±4.0 wt.-%, and in particular 48±2.0 wt.-%; in each case relative to the total weight of the one or more saccharides and relative to the total weight of the tablet.

In comparative embodiments, the one or more saccharides comprise or essentially consist of lactose.

The lactose may be present in form of lactose monohydrate, crystalline alpha-lactose monohydrate, spray-dried lactose, or anhydrous lactose. Preferably, the lactose is anhydrous lactose.

The one or more saccharides comprise or essentially consist of a mixture of glucose and maltose, optionally together with one or more oligosaccharides. Such mixtures are commercially available and commonly referred to as *"dextrates".* Preferably, the one or more saccharides essentially consist of a mixture of glucose and maltose, optionally together with one or more oligosaccharides, and in addition to the glucose, maltose and optionally present one or more oligosaccharides, the core preferably contains neither any other monosaccharide nor any other disaccharide.

Preferably, the core of the tablet according to the invention does not contain lactose; preferably the tablet according to the invention does not contain lactose at all.

Preferably, the mixture of glucose, maltose, and optionally one or more oligosaccharides contains glucose as the predominant ingredient, followed by maltose, whereas the optionally present one or more oligosaccharides are present at comparatively low content. Preferably, the total content of glucose and maltose amounts to at least 99 wt.-%, relative to the total weight of the mixture.

For the purpose of the specification, the mixture is dextrates, i.e. a is a purified mixture of saccharides resulting from the controlled enzymatic hydrolysis of starch. It may be either hydrated or anhydrous. Its dextrose equivalent is typically not less than 93.0% and not more than 99.0%, calculated on the dried basis. In addition to dextrose, dextrates typically contains 3-5 wt.-% maltose and higher oligo- or polysaccharides.

In preferred embodiments, dextrates according to the invention comprises about 95 wt.-% glucose monohydrate.

Preferably, the one or more saccharides comprise glucose, wherein the weight content of the glucose is at least 10 wt.-%; preferably at least 15 wt.-%, more preferably at least 20 wt.-%, still more preferably at least 25 wt.-%, yet more preferably at least 30 wt.-%, even more preferably at least 35 wt.-%, most preferably at least 40 wt.-%, and in particular at least 45 wt.-%; in each case relative to the total weight of the tablet.

Preferably, the one or more saccharides comprise glucose, wherein the weight content of the glucose is at most 90 wt.-%; preferably at most 85 wt.-%, more preferably at most 80 wt.-%, still more preferably at most 75 wt.-%, yet more preferably at most 70 wt.-%, even more preferably at most 65 wt.-%, most preferably at most 60 wt.-%, and in particular at most 55 wt.-%; in each case relative to the total weight of the tablet.

Preferably, the one or more saccharides comprise glucose, wherein the weight content of the glucose is within the range of from 0.1 to 80 wt.-%, preferably 0.1 to 70 wt.-%, more preferably 0.1 to 60 wt.-%, still more preferably 0.1 to 50 wt.-%, yet more preferably 0.1 to 40 wt.-%, most preferably 0.1 to 30 wt.-%; in each case relative to the total weight of the tablet.

Preferably, the one or more saccharides comprise glucose and wherein the weight content of the glucose is within the range of from 1.0 to 60 wt.-%, preferably 1.0 to 50 wt.-%; in each case relative to the total weight of the tablet.

Preferably, the one or more saccharides comprise glucose and wherein the weight content of the glucose is within the range of 48±16 wt.-%; preferably 48±14 wt.-%, more preferably 48±12 wt.-%, still more preferably 48±10 wt.-%, yet more preferably 48±8.0 wt.-%, even more preferably 48±6.0 wt.-%, most preferably 48±4.0 wt.-%, and in particular 48±2.0 wt.-%; in each case relative to the total weight of the tablet.

Preferably, the dextrates comprise maltose, whereas the weight content of maltose is at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Preferably, the one or more saccharides comprise maltose and wherein the weight content of the maltose is at least 0.1 wt.-%; preferably at least 0.25 wt.-%, more preferably at least 0.5 wt.-%, still more preferably at least 0.75 wt.-%, yet more preferably at least 1.0 wt.-%, even more preferably at least 1.25 wt.-%, most preferably at least 1.5 wt.-%, and in particular at least 1.75 wt.-%; in each case relative to the total weight of the tablet.

Preferably, the one or more saccharides comprise maltose and wherein the weight content of the maltose is at most 10 wt.-%; preferably at most 9.0 wt.-%, more preferably at most 8.0 wt.-%, still more preferably at most 7.0 wt.-%, yet more preferably at most 6.5 wt.-%, even more preferably at most 6.0 wt.-%, most preferably at most 5.5 wt.-%, and in particular at most 5.0 wt.-%; in each case relative to the total weight of the tablet.

Preferably, the one or more saccharides comprise maltose and wherein the weight content of the maltose is within the range of 4.0±3.2 wt.-%; preferably 4.0±2.8 wt.-%, more preferably 4.0±2.4 wt.-%, still more preferably 4.0±2.0 wt.-%, yet more preferably 4.0±1.6 wt.-%, even more preferably 4.0±1.2 wt.-%, most preferably 4.0±0.8 wt.-%, and in particular 4.0±0.4 wt.-%; in each case relative to the total weight of the tablet.

Preferably, the one or more saccharides, i.e. dextrates, comprise isomaltose, whereas the weight content of isomaltose is preferably at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Preferably, the one or more saccharides, i.e. dextrates, comprise maltotriose, whereas the weight content of maltotriose is preferably at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Preferably, the one or more saccharides, i.e. dextrates, comprise isomaltotriose, whereas the weight content of isomaltotriose is preferably at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Preferably, the one or more saccharides, i.e. dextrates, comprise panose, whereas the weight content of panose is preferably at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Preferably, the one or more saccharides, i.e. dextrates, comprise maltotetraose, whereas the weight content of maltotetraose is preferably at least 0.01 wt.-%, more preferably at least 0.02 wt.-%, still more preferably at least 0.03 wt.-%, yet more preferably 0.04 wt.-%, even more preferably at least 0.05 wt.-%, most preferably at least 0.06 wt.-%, and in particular at least 0.07 wt.-%, in each case relative to the total weight of the tablet.

Edoxaban has the chemical names N1-(5-Chloro-2-pyridinyl)-N2-[(1S,2R,4S)-4-[(dimethylamino)carbonyl]-2-[[(4,5,6,7-tetrahydro-5-methylthiazolo[5,4-c]pyridin-2-yl)carbonyl]amino]cyclohexyl]-ethanediamide or alternatively N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide (CAS 480449-70-5, C₂₄H₃₀ClN₇O₄S).

Edoxaban has the following chemical structure: 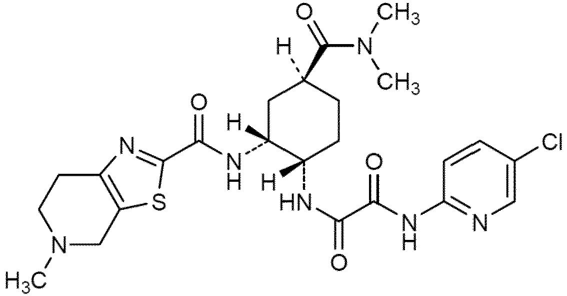 (WHO Drug Information, Vol. 22, No. 2, 2008, page 135).

Edoxaban can be in the form of base or a pharmaceutically acceptable salt thereof, or a solvate thereof. Preferably, the Edoxaban, pharmaceutically acceptable salt and/or solvate thereof is Edoxaban tosilate, i.e. 4-methylbenzenesulfonate (1:1) (CAS 480449-71-6).

Preferably, the Edoxaban tosilate is Edoxaban tosilate monohydrate.

Preferably, the Edoxaban tosilate monohydrate comprises or essentially consists of polymorph form I, form II or mixtures thereof (see e.g. EP 2 548 879 A1). Preferably, Edoxaban tosilate is present as form I.

Edoxaban may be characterized with regard to its X-ray powder diffraction patterns (e.g. obtained by a PANalytical X'Pert PRO diffractometer using CuKa radiation of 1.541874 A).

Polymorph form I of Edoxaban tosilate monohydrate has a powder x-ray diffraction pattern as a diffraction angle (20 (°)) in powder x-ray diffraction obtained using Cu-Kα rays and characteristic peaks at diffraction angles (20 (°)) of 5.38±0.2, 8.08±0.2, 10.8±0.2, 13.5±0.2, 15.0±0.2, 16.9±0.2, 17.6±0.2, 20.5±0.2, 21.1±0.2, 22.7±0.2, 23.5±0.2, 26.0±0.2, 27.3±0.2, 27.6±0.2, and 30.0±0.2 (°). Crystals of polymorph form I of Edoxaban tosilate monohydrate can be produced by a method described in e.g. WO 03/000657, WO 03/000680, WO 03/016302, WO 04/058715, WO 05/047296, WO 07/032498, WO 08/129846, WO 08/156159, and Japanese Patent Laid-Open No. 2010-254615. Preferably, polymorph form I of Edoxaban tosilate monohydrate has any feature selected from the group consisting of the following (i) to (iii):
(i) a DTA profile having two endothermic peaks at approximately 250°C to approximately 270°C;
(ii) an infrared absorption spectrum comprising any one absorption band selected from the group consisting of 3344±5, 1675±2, 1614±2, 1503±2, 1222±1, 1171±1, 1033±1, 1012±1, 843±1, 825±1, and 802±1 (cm⁻¹); and/or
(iii) a melting point (decomp.) of approximately 246°C to approximately 250°C.

While it is contemplated that the tablet according to the invention may contain combinations of two or more pharmacologically active ingredients with one another, the Edoxaban, physiologically acceptable salt and/or solvate thereof is preferably the only, i.e. the sole pharmacologically active ingredient that is contained in the tablet according to the invention.

Preferably, the Edoxaban, pharmaceutically acceptable salt and/or solvate thereof is characterized by an average particle size within the range of from 1 to 200 µm, preferably 1 to 100 µm, more preferably from 1 to 50 µm, even more preferably from 1 to 30 µm.

The term "average particle size" as used herein preferably refers to the volume mean diameter of particles. The diameter and volume mean diameter can be determined by laser light scattering using e.g. a Malvern-Mastersizer Apparatus MS 2000, MS 3000 or equivalent with "wet" dispersion unit. Particle sizes are determined by measuring the angular distribution of laser light scattered by a homogeneous suspension of particles. The particles to be subjected to particle size measurement are first suspended in an appropriate non-polar dispersant and then subjected to size determination in a Malvern Mastersizer instrument. Preferably, the particle size is determined in accordance with Eur. Ph. 10.0, *2.9.31 "Particle size analysis by laser diffraction".*

Alternatively, the "average particle size" may also be determined by other methods, i.e. analysis of SEM (scanning electron microscope) images. The image analysis process for determining the size distribution of the particles essentially consists of a visual count of all the crystals appearing in an SEM image containing at least 200 particles. The size determined for each particle is that of the largest cross section of said particle concerned.

Preferably, the Edoxaban, pharmaceutically acceptable salt and/or solvate thereof is contained at a dose of at least 10 mg; preferably at least 20 mg, more preferably at least 30 mg, still more preferably at least 40 mg, yet more preferably at least 45 mg, even more preferably at least 50 mg, most preferably at least 55 mg, and in particular at least 60 mg; in each case expressed as equivalent weight of the non-salt and non-solvate form of Edoxaban.

Preferably, the weight content of the Edoxaban, pharmaceutically acceptable salt and/or solvate thereof is at least 4.0 wt.-%; preferably at least 6.0 wt.-%, more preferably at least 8.0 wt.-%, still more preferably at least 10 wt.-%, yet more preferably at least 12 wt.-%, even more preferably at least 14 wt.-%, most preferably at least 16 wt.-%, and in particular at least 18 wt.-%; in each case relative to the given weight of the Edoxaban, pharmaceutically acceptable salt and/or solvate thereof and relative to the total weight of the tablet.

Preferably, the weight content of the Edoxaban, pharmaceutically acceptable salt and/or solvate thereof is at most 55 wt.-%; preferably at most 50 wt.-%, more preferably at most 45 wt.-%, still more preferably at most 40 wt.-%, yet more preferably at most 35 wt.-%, even more preferably at most 30 wt.-%, most preferably at most 25 wt.-%, and in particular at most 20 wt.-%; in each case relative to the given weight of the Edoxaban, pharmaceutically acceptable salt and/or solvate thereof and relative to the total weight of the tablet.

Preferably, the weight content of the Edoxaban, pharmaceutically acceptable salt and/or solvate thereof is within the range of 19±16 wt.-%; preferably 19±14 wt.-%, more preferably 19±12 wt.-%, still more preferably 19±10 wt.-%, yet more preferably 19±8.0 wt.-%, even more preferably 19±6.0 wt.-%, most preferably 19±4.0 wt.-%, and in particular 48±2.0 wt.-%; in each case relative to the given weight of the Edoxaban, pharmaceutically acceptable salt and/or solvate thereof and relative to the total weight of the tablet.

The core of the tablet according to the invention preferably comprises one or more binders.

Preferably, the one or more binders comprise or consist of
(i) cellulose or nonionic cellulose ethers; such as crystalline cellulose, methyl cellulose, ethyl cellulose, hydroxyethyl cellulose, hydroxypropyl cellulose, hydroxyethylmethyl cellulose, hydroxypropylmethyl cellulose (e.g. 2208, 2906, or 2910);
(ii) cellulose esters; such as cellulose acetate phthalate, or hydroxypropylmethyl cellulose phthalate;
(iii) starch and starch derivatives except pregelatinized starch and sodium starch glycolate; such as starch, flour, wheat starch, rice powder, rice starch, cornstarch, potato starch, or hydroxypropyl starch;
(iv) gums; such as alginic acid, sodium alginate, propylene glycol alginate ester, agar, gum arabic, arabinogalactan, tragacanth gum, locust bean gum, acacia gum, xanthan gum, pectin, gelatin, purified gelatin, or hydrolyzed gelatin powder;
(v) glycerin and polyethylene glycol; such as concentrate glycerin, or Macrogol (e.g. 4000, 6000, or 20000);
(vi) silicious materials; such as hydrous silicon dioxide, highly disperse silicon dioxide, light anhydrous silicic acid, or synthetic aluminum silicate;
(vii) vinyl polymers except crospovidone; such as carboxyl vinyl polymer, polyvinyl acetate, povidone, copovidone, copolydone, high molecular weight polyvinylpyrrolidone, polyvinyl alcohol (completely saponified product), polyvinyl alcohol (partially saponified product), vinylpyrrolidone-vinyl acetate copolymer, or sodium polyacrylate;
(viii) sodium polyphosphate;
or mixtures thereof.

Preferably, the one or more binders comprise or consist of hydroxypropyl cellulose.

Preferably, the weight content of the one or more binders is at least 0.7 wt.-%; preferably at least 0.8 wt.-%, more preferably at least 0.9 wt.-%, still more preferably at least 1.0 wt.-%, yet more preferably at least 1.1 wt.-%, even more preferably at least 1.2 wt.-%, most preferably at least 1.3 wt.-%, and in particular at least 1.4 wt.-%; in each case relative to the total weight of the one or more binders and relative to the total weight of the tablet.

Preferably, the weight content of the one or more binders is at most 8.0 wt.-%; preferably at most 7.0 wt.-%, more preferably at most 6.0 wt.-%, still more preferably at most 5.0 wt.-%, yet more preferably at most 4.5 wt.-%, even more preferably at most 4.0 wt.-%, most preferably at most 3.5 wt.-%, and in particular at most 3.0 wt.-%; in each case relative to the total weight of the one or more binders and relative to the total weight of the tablet.

Preferably, the weight content of the one or more binders is within the range of 2.2±2.0 wt.-%; preferably 2.2±1.8 wt.-%, more preferably 2.2±1.6 wt.-%, still more preferably 2.2±1.4 wt.-%, yet more preferably 2.2±1.2 wt.-%, even more preferably 2.2±1.0 wt.-%, most preferably 2.2±0.9 wt.-%, and in particular 2.2±0.8 wt.-%; in each case relative to the total weight of the one or more binders and relative to the total weight of the tablet.

The core of the tablet according to the invention preferably comprises one or more disintegrants.

Preferably, the one or more disintegrants comprise or consist of pregelatinized starch, crospovidone, sodium starch glycolate, carmellose, carmellose sodium, croscarmellose, croscarmellose sodium, or mixtures thereof.

Preferably, the one or more disintegrants comprise or consist of pregelatinized starch, crospovidone, or a mixture thereof.

Preferably, the one or more disintegrants comprise or consist of a mixture of pregelatinized starch, and crospovidone. Crospovidone is swellable in water but not soluble. Thus, crospovidone is no water soluble vinylpyrrolidone. This is a distinction of the present invention from the teaching of EP 3 177 290 A1, which requires the use of water soluble vinylpyrrolidones.

Preferably, the relative weight ratio of the pregelatinized starch to the crospovidone is within the range of from 7:1 to 1:1; preferably 6:1 to 2:1, more preferably 5:1 to 3:1.

Preferably, the weight content of the one or more disintegrants is at least 10 wt.-%; preferably at least 12 wt.-%, more preferably at least 14 wt.-%, still more preferably at least 16 wt.-%, yet more preferably at least 18 wt.-%, even more preferably at least 20 wt.-%, most preferably at least 22 wt.-%, and in particular at least 24 wt.-%; in each case relative to the total weight of the one or more disintegrants and relative to the total weight of the tablet.

Preferably, the weight content of the one or more disintegrants is at most 40 wt.-%; preferably at most 38 wt.-%, more preferably at most 36 wt.-%, still more preferably at most 34 wt.-%, yet more preferably at most 32 wt.-%, even more preferably at most 30 wt.-%, most preferably at most 28 wt.-%, and in particular at most 26 wt.-%; in each case relative to the total weight of the one or more disintegrants and relative to the total weight of the tablet.

Preferably, the weight content of the one or more disintegrants is within the range of 25±18 wt.-%; preferably 25±16 wt.-%, more preferably 25±14 wt.-%, still more preferably 25±12 wt.-%, yet more preferably 25±10 wt.-%, even more preferably 25±8.0 wt.-%, most preferably 25±6.0 wt.-%, and in particular 25±4.0 wt.-%; in each case relative to the total weight of the one or more disintegrants and relative to the total weight of the tablet.

The core of the tablet according to the invention preferably comprises one or more lubricants.

Preferably, the one or more lubricants comprise or consist of magnesium stearate, calcium stearate, stearic acid, stearyl alcohol, polyoxyl stearate, calcium carbonate, magnesium oxide, talc, bees wax, carnauba wax, aluminum hydroxide, fumaric acid, or mixtures thereof; preferably magnesium stearate.

Preferably, the weight content of the one or more lubricants is at least 0.1 wt.-%; preferably at least 0.15 wt.-%, more preferably at least 0.2 wt.-%, still more preferably at least 0.3 wt.-%, yet more preferably at least 0.4 wt.-%, even more preferably at least 0.5 wt.-%, most preferably at least 0.6 wt.-%, and in particular at least 0.7 wt.-%; in each case relative to the total weight of the one or more lubricants and relative to the total weight of the tablet.

Preferably, the weight content of the one or more lubricants is at most 7.0 wt.-%; preferably at most 2.5 wt.-%, more preferably at most 2.0 wt.-%, still more preferably at most 1.8 wt.-%, yet more preferably at most 1.6 wt.-%, even more preferably at most 1.4 wt.-%, most preferably at most 1.2 wt.-%, and in particular at most 1.0 wt.-%; in each case relative to the total weight of the one or more lubricants and relative to the total weight of the tablet.

Preferably, the weight content of the one or more lubricants is within the range of 0.8±0.7 wt.-%; preferably 0.8±0.6 wt.-%, more preferably 0.8±0.5 wt.-%, still more preferably 0.8±0.4 wt.-%, yet more preferably 0.840.3 wt.-%, even more preferably 0.8±0.2 wt.-%, most preferably 0.8±0.15 wt.-%, and in particular 0.8±0.1 wt.-%; in each case relative to the total weight of the one or more lubricants and relative to the total weight of the tablet.

Preferably, the tablet according to the invention comprises a coating encapsulating the core, wherein the coating is preferably a non-functional coating.

Preferably, the tablet according to the invention comprises a coating encapsulating the core, wherein the coating comprises polyethylene glycol, hydroxypropylmethylcellulose, talc, or mixtures thereof.

Preferably, the tablet according to the invention comprises a coating encapsulating the core, wherein the weight content of the coating is at least 1.0 wt.-%; preferably at least 1.5 wt.-%, more preferably at least 2.0 wt.-%, still more preferably at least 2.5 wt.-%, yet more preferably at least 3.0 wt.-%, even more preferably at least 3.5 wt.-%, most preferably at least 4.0 wt.-%, and in particular at least 4.5 wt.-%; in each case relative to the total weight of the coating and relative to the total weight of the tablet.

Preferably, the tablet according to the invention comprises a coating encapsulating the core, wherein the weight content of the coating is at most 8.5 wt.-%; preferably at most 8.0 wt.-%, more preferably at most 7.5 wt.-%, still more preferably at most 7.0 wt.-%, yet more preferably at most 6.5 wt.-%, even more preferably at most 6.0 wt.-%, most preferably at most 5.5 wt.-%, and in particular at most 5.0 wt.-%; in each case relative to the total weight of the coating and relative to the total weight of the tablet.

Preferably, the tablet according to the invention comprises a coating encapsulating the core, wherein the weight content of the coating is within the range of 5.0±4.0 wt.-%; preferably 5.0±3.5 wt.-%, more preferably 5.0±3.0 wt.-%, still more preferably 5.0±2.5 wt.-%, yet more preferably 5.0±2.0 wt.-%, even more preferably 5.0±1.5 wt.-%, most preferably 5.0±1.0 wt.-%, and in particular 5.0±0.5 wt.-%; in each case relative to the total weight of the coating and relative to the total weight of the tablet.

Preferably, the tablet according to the invention has an average hardness of at least 80 N; preferably at least 85 N, more preferably at least 90 N, still more preferably at least 95 N, even more preferably at least 100 N, most preferably at least 105 N, and in particular at least 110 N.

Preferably, the tablet according to the invention is wet granulated.

Preferably, under *in vitro* conditions in accordance with Ph. Eur., paddle method, 50 rpm, 37°C, 900 mL phosphate buffer solution without enzyme, pH 6.8, the tablet according to the invention exhibits an average percent dissolution profile
- at 30 minutes of at least 60%, preferably at least 70%; and
- at 60 minutes of at least 70%, preferably at least 80%;
in each case after the start of the dissolution test and in each case relative to the total amount of Edoxaban that was originally contained in the tablet.

Preferably, under *in vitro* conditions in accordance with Ph. Eur., paddle method, 50 rpm, 37°C, 900 mL phosphate buffer solution without enzyme, pH 6.8, the tablet according to the invention exhibits an average percent dissolution profile at 45 minutes of at least 70%, preferably at least 75%; more preferably at least 80%; in each case after the start of the dissolution test and in each case relative to the total amount of Edoxaban that was originally contained in the tablet.

Preferably, under *in vitro* conditions in accordance with Ph. Eur., paddle method, 50 rpm, 37°C, 900 mL acetic acid sodium acetate buffer, pH 4.5, the tablet according to the invention exhibits an average percent dissolution profile at 30 minutes of at least 85%, preferably at least 90%; and in each case after the start of the dissolution test and in each case relative to the total amount of Edoxaban that was originally contained in the tablet.

Preferably, under *in vitro* conditions in accordance with Ph. Eur., paddle method, 50 rpm, 37°C, 900 mL acetic acid sodium acetate buffer, pH 4.5, the tablet according to the invention exhibits an average percent dissolution profile at 30 minutes of at least 85%, preferably at 15 minutes of at least 85%; in each case after the start of the dissolution test and in each case relative to the total amount of Edoxaban that was originally contained in the tablet.

Preferably, the tablets according to the invention disintegrate within 12 minutes, preferably within 10 minutes, more preferably within 8, even more preferably within 6 minutes, when disintegration time is determined according to Ph. Eur. 10.0, 2.9.1 *"Disintegration of tablets and capsules",* Test A, with disc, in purified water, 750 ml, at 37 degC.

Preferably, the tablets according to the invention disintegrate within 20 minutes, preferably within 15 minutes, more preferably within 12, when disintegration time is determined according to Ph. Eur. 10.0, 2.9.1 *"Disintegration of tablets and capsules",* Test A, without disc, in purified water, 750 ml, with the distinction that the temperature is measured at 25 degC.

Preferably, the tablet according to the invention has a total weight within the range of 420±375 mg, preferably 420±350 mg, more preferably 420±300 mg, still more preferably 420±250 mg, yet more preferably 420±200 mg, even more preferably 420±150 mg, most preferably 420±100 mg, and in particular 420±50 mg.

The shape of the tablet according to the invention is not particularly limited and may be round or oblong. Preferably, the tablet according to the invention is a round tablet.

The size of the tablet according to the invention is not particularly limited. Preferably, the tablet according to the invention has a diameter within the range of 11±4.0 mm; preferably 11±3.0 mm, more preferably 11±2.0 mm, most preferably 11±1.0 mm.

The thicknes of the tablet according to the invention is not particularly limited. Preferably, the tablet thicknes according to the invention is within the range of 4.5±2.0 mm, preferably 4.5±1.0 mmm more preferably 4.5±0.5 mm.

Polymorphic form of the active substance does not change in the formulation, when stored at 45 degC/75%RH, for at least 3 months, preferably 6 months, more preferably 12 months.

The tablet according to the invention is useful for the prevention and/or treatment of a disease, disorder or condition that can be prevented and/or treated by inhibition of activated blood coagulation factor X (FXa).

Thus, another aspect of the invention relates to the tablet according to the invention as described above for use in the prevention and/or treatment of disease, disorder or condition that can be prevented and/or treated by inhibition of activated blood coagulation factor X (FXa). Another aspect of the invention relates to the use of Edoxaban, a pharmaceutically acceptable salt and/or solvate thereof for the manufacture of a tablet according to the invention as described above for the prevention and/or treatment of disease, disorder or condition that can be prevented and/or treated by inhibition of activated blood coagulation factor X (FXa). Another aspect of the invention relates to a method for the prevention and/or treatment of a disease, disorder or condition that can be prevented and/or treated by inhibition of activated blood coagulation factor X (FXa), comprising the administration of a tablet according to the invention as described above to a subject in need thereof.

Preferably, the tablet according to the invention is administered orally once daily or twice daily.

Preferably, the tablet according to the invention is for use as anticoagulant in the prevention and/or treatment of thrombosis, thrombotic diseases or embolism.

Preferably, the tablet according to the invention is for use in the prevention and/or treatment of cerebral infarction, cerebral embolism, pulmonary infarction, pulmonary embolism, myocardial infarction, angina pectoris, thrombus and/or embolism accompanying nonvalvular atrial fibrillation, deep vein thrombosis, deep vein thrombosis after surgery, thrombosis after prosthetic valve/joint replacement, thromboembolism after total hip replacement, thromboembolism after total knee replacement, thromboembolism after hip fracture surgery, thrombosis and/or reocclusion after revascularization, Buerger's disease, disseminated intravascular coagulation syndrome, systemic inflammatory response syndrome, multiple organ dysfunction syndrome thrombosis at the time of extracorporeal circulation, or blood coagulation at the time of blood collection.

Preferably, the tablet according to the invention is for use in the prevention of stroke and systemic embolism in adult patients with nonvalvular atrial fibrillation with one or more risk factors; preferably selected from congestive heart failure, hypertension, age ≥ 75 years, diabetes mellitus, prior stroke and transient ischemic attack.

Preferably, the tablet according to the invention is for use in the treatment of deep vein thrombosis and/or pulmonary embolism, or for use in the prevention of recurrent deep vein thrombosis and/or pulmonary embolism in adults.

Preferably, the tablet according to the invention is for use in the prevention of stroke and/or systemic embolism, wherein one or more tablets containing a total dose of 60 mg expressed as equivalent weight of the non-salt and non-solvate form of Edoxaban is or are administered once daily.

Preferably, the tablet according to the invention is for use in the treatment of venous thromboembolism including deep vein thrombosis and/or pulmonary embolism, or prevention of recurrent venous thromboembolism, wherein following initial use of heparin, one or more tablets containing a total dose of 60 mg expressed as equivalent weight of the non-salt and non-solvate form of Edoxaban is or are administered once daily.

Preferably, the tablet according to the invention is for use in the prevention or treatment of nonvalvular atrial fibrillation and/or venous thromboembolism, wherein one or more tablets containing a total dose of 30 mg Lixiana expressed as equivalent weight of the non-salt and non-solvate form of Edoxaban is or are administered once daily in patients with one or more of the following clinical factors:
A) moderate or severe renal impairment (creatinine clearance: 15-50 ml/min);
B) low body weight ≤60 kg;
C) concomitant use of one or more of the following P-glycoprotein (P-gp) inhibitors: ciclosporine, dronedarone, erythromycin, ketoconazole.

The subjects to be treated with the tablet according to the invention are preferably animals, more preferably humans, still more preferably adults.

Another aspect of the invention relates to a process for the preparation of a tablet according to the invention as described above, preferably comprising wet granulation.

Preferably, wet granulation includes an aqueous medium.

Preferably, granulation involves high shear granulation (mixing granulation).

High shear-granulation is a technology known to the skilled person and is distinguished from other granulation methods such as drum granulation, pan granulation and fluidized-bed granulation in the higher shearing force that is applied to the material.

Preferably, the process according to the invention involves high shear granulation by means of high shear granulators. High-shear granulation may involve high-speed stirring granulation. Preferably, stirring is performed by stirring blades that granulate the mixture by kneading.

In a preferred embodiment, high shear granulation is performed by means of a horizontal high shear granulator. In another preferred embodiment, high shear granulation is performed by means of a vertical high shear granulator.

High shear granulators according to the invention use an impeller to vigorously agitate the powder and produce high-density granules. Preferably, the impellers rotate at a speed within the range of from 100 to 1500 rpm. Preferably, the high shear granulator is additionally equipped with a secondary smaller impeller, sometimes referred to as *"chopper",* which preferably rotates at higher speed, preferably within the range of from 1000 to 2000 rpm. Preferably, the high shear granulator is equipped with a feed unit that allows for spraying a liquid onto the material being granulated.

Preferably, the granules are prepared by means of a high-shear granulator under conditions keeping the water content of the granules during granulation higher than 10%. This is a distinction of the present invention over the teaching of EP 2 548 556 which requires that the maximum water content of the granules during granulation must be kept at 10% or less.

In preferred embodiments, the process according to the invention comprises the steps of
(a) providing a composition comprising
   - Edoxaban, a pharmaceutically acceptable salt and/or solvate thereof;
   - one or more saccharides comprising or essentially consisting of a mixture of glucose and maltose, optionally together with one or more oligosaccharides, being dextrates;
   - optionally, one or more binders and
   - optionally, one or more disintegrants;
(b) providing a granulation liquid comprising a solvent and optionally one or more binders;
(c) mixing the composition provided in step (a) by means of a mixing device thereby obtaining a granulation mixture;
(d) wet granulating the granulation mixture obtained in step (c) by means of a granulating device while adding the granulation liquid provided in step (b) and optionally a solvent thereby obtaining wet granules;
(e) drying the wet granules obtained in step (d) by means of a drying device thereby obtaining dried granules;
(f) optionally, mixing the dried granules with one or more lubricants thereby obtaining lubricated dried granules;
(g) compressing the dried granules obtained in step (e) or the lubricated dried granules obtained in step (f) by means of a tableting device thereby obtaining tablet cores; and
(h) optionally, coating the tablet cores obtained in step (g) by means of a coating device.

In step (a) of the process according to the invention, a composition is provided which comprises
- Edoxaban, a pharmaceutically acceptable salt and/or solvate thereof;
- one or more saccharides comprising or essentially consisting of a mixture of glucose and maltose, optionally together with one or more oligosaccharides, being dextrates;
- optionally, one or more binders, and
- optionally, one or more disintegrants.

Preferably, all ingredients are weighed and combined with one another.

In step (b) of the process according to the invention, a granulation liquid is provided which comprises a solvent and optionally one or more binders.

Preferably, in step (b) the solvent is an aqueous liquid, preferably water.

In step (c) of the process according to the invention, the composition provided in step (a) is mixed by means of a mixing device thereby obtaining a granulation mixture.

Preferably, in step (c) the mixing device is a high shear mixer.

Preferably, the mixing device in step (c) and the granulating device in step (d) are one and the same device.

In step (d) of the process according to the invention, the granulation mixture obtained in step (c) is wet granulated by means of a granulating device while adding the granulation liquid provided in step (b) and optionally a solvent thereby obtaining wet granules.

Preferably, in step (d) the amount of added granulation liquid and optional solvent is such that the loss on drying of the wet granules is more than 10 wt.-%; preferably at least 15 wt.-%, more preferably at least 17.5 wt.-%, still more preferably at least 20 wt.-%, yet more preferably at least 22.5 wt.-%, even more preferably at least 25 wt.-%, most preferably at least 27.5 wt.-%, and in particular at least 30 wt.-%; in each case measured after heating a 2 g sample of wet granules at 85°C through absorption of IR radiation from a halogen radiator until weight constancy.

Preferably, the granulation liquid comprises water. Preferably, the water content (%) of the granules during granulation is comparatively high. For the purpose of the specification, the "water content (%) of the granules during granulation" refers to the value of a water content (wt.-%) of the mixture and/or granules (including the mixture at the start of granulation) obtained from the mixing of the Edoxaban, etc., an excipient, and other appropriate additives to the completion of granulation. In this context, the "water content (%) of the granules" is the value measured by a loss on drying method. For example, the water content (%) of the granules can be calculated by evaporating water from collected granules by heating and determining the proportion of the amount of water evaporated by this heating to 2 g of the collected granules. For example, when the collected granules are m (g) and become n (g) by heating, water is m-n (g) and the water content (%) of the granules can be calculated according to (m - n) / m x 100.

Preferably, in step (d) the solvent is an aqueous liquid, preferably water.

Preferably, in step (d) the granulating device is a high shear granulator as defined above.

Preferably, in step (d) the adding is performed by spraying the granulation liquid provided in step (b) and optionally the solvent onto the granulation mixture.

Preferably, in step (d) the granulation liquid is added to the granulation mixture first, followed by adding the solvent.

In step (e) of the process according to the invention, the wet granules obtained in step (d) are dried by means of a drying device thereby obtaining dried granules.

Preferably, in step (e) the drying is performed such that after the drying, the additional loss on drying of the dried granules is at most 12 wt.-%; preferably at most 11 wt.-%, more preferably at most 10 wt.-%, still more preferably at most 9.5 wt.-%, yet more preferably at most 8.0 wt.-%, even more preferably at most 7.5 wt.-%, most preferably at most 7.0 wt.-%, and in particular at least 6.5 wt.-%; in each case measured after heating a 2 g sample of dried granules at 85°C through absorption of IR radiation from a halogen radiator until weight constancy.

Preferably, in step (e) the drying is performed such that after the drying, the additional loss on drying of the dried granules is at least 0.5 wt.-%; preferably at least 1.0 wt.-%, more preferably at least 2.0 wt.-%, still more preferably at least 3.0 wt.-%, yet more preferably at least 4.0 wt.-%, even more preferably at least 5.0 wt.-%, most preferably at least 5.5 wt.-%, and in particular at least 6.0 wt.-%; in each case measured after heating a 2 g sample of dried granules at 85°C through absorption of IR radiation from a halogen radiator until weight constancy.

In optional step (f) of the process according to the invention, the dried granules are mixed with one or more lubricants thereby obtaining lubricated dried granules.

In step (g) of the process according to the invention, the dried granules obtained in step (e) or the lubricated dried granules obtained in step (f) are compressed by means of a tableting device thereby obtaining tablet cores.

Preferably, in step (g) compression is performed at a pressure of at least 5 kN, preferably at least 6 kN, more preferably at least 7 kN.

In optional step (h) of the process according to the invention, the tablet cores obtained in step (g) are coated by means of a coating device. Preferably, a coating solution or suspension is sprayed onto the tablet cores and dried. Preferably, the coating solution or suspension is aqueous.

Another aspect of the invention relates to a tablet obtainable by the process according to the invention as described above.

The following examples further illustrate the invention but are not to be construed as limiting its scope. Examples 2 and 9 to 14 are Inventive Examples. Examples 1 and 3 to 8 are Comparative Examples.

### Examples 1 and 2:

Edoxaban was characterized with regard to X-ray powder diffraction patterns (obtained by a PANalytical X'Pert PRO diffractometer using CuKa radiation of 1.541874 A). Polymorphic form of Edoxaban as used in Example 1 and 2 was characterized by a powder X- ray diffraction pattern and corresponds to form I according to EP 2 548 879 B1.

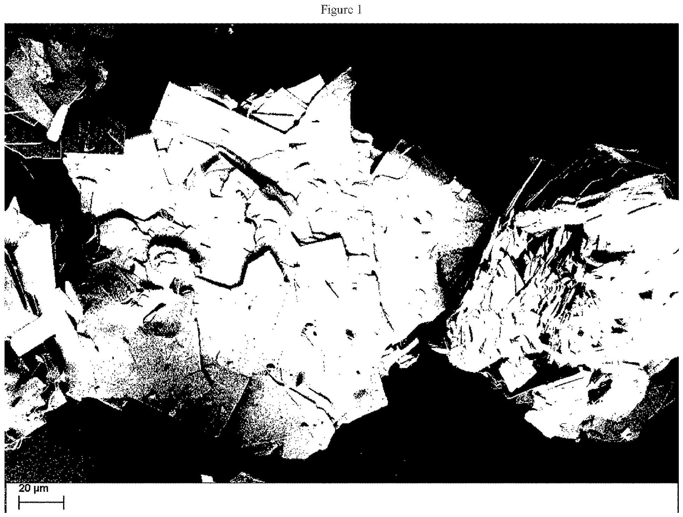

SEM images of Edoxaban as used in Example 1 and 2 were acquired under high vacuum on a scanning electron microscope CarIZeiss Ultra Plus. The accelerating voltage was 1 -2 kV and the working distance was 2.1-5.3 mm. SEM image of the product is shown in Figure 1.

Tablets were prepared by wet granulation from the following compositions:

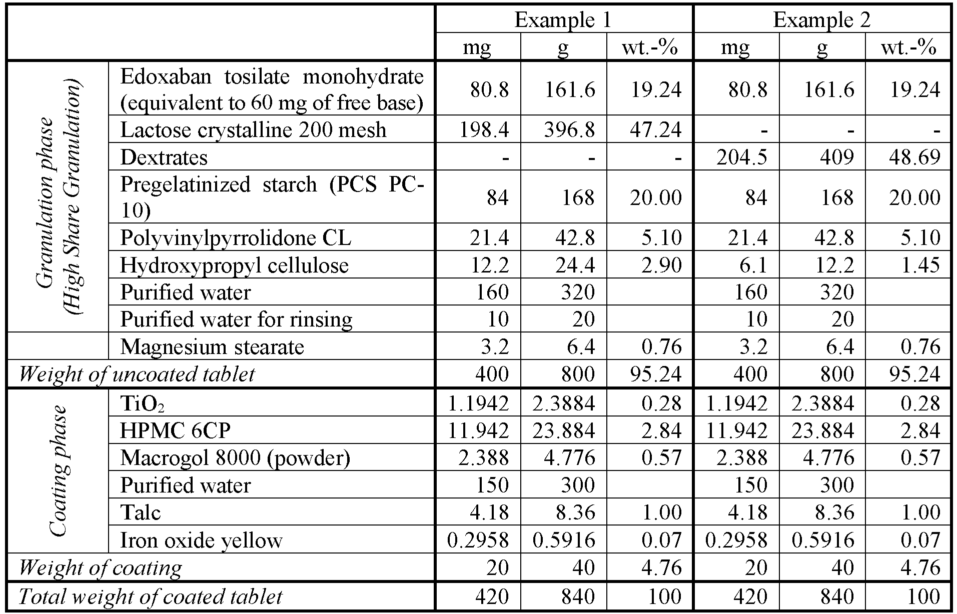

Ingredients of the granulation phase as shown in the above table, except for hydroxypropyl cellulose and magnesium stearate, were mixed for 2 minutes. Then, the mixture was granulated for approximatelly 9 minutes by use of a high shear mixer while spraying of aqueous hydroxypropyl cellulose solution thereon. Additionally, purified water for rinsing the spraying system was sprayed thereon, such that the loss on drying of the wet granules was 30 ± 2 percent by weight for Example 1 (with lactose) and 33 ± 2 for Example 2 (with dextrates), measured after heating a 2 g sample of wet granules at 85°C through absorption of IR radiation from a halogen radiator.

Then, the wet granules were dried at inlet air temperature of 65°C until the granulate temperature reached 41°C, such that the loss on drying of the dried granules was 1 to 3 percent by weight for Example 1 and 6 to 8 percent by weight for Example 2, measured after heating a 2 g sample of dried granules at 85°C through absorption of IR radiation from a halogen radiator.

The thus produced wet granules were mixed with magnesium stearate and compressed into tablets (round, 11 mm in diameter, compression pressure: approximately 11 kN) by use of a tableting machine (Kilian Pressima). In this way, uncoated tablets having average hardness of 111 N were obtained.

Furthermore, ingredients of the coating phase, as shown in the above table were mixed with water to obtain an aqueous suspension. Film-coated tablets were produced by use of an aqueous suspension and a pan coater (Glatt GC II).

Tablets were exposed to 40 °C/75% RH. Polymorphic form I of Edoxaban (cordesponding to Form I according to EP 2 548 879 B1), was determined in the tablets at the time 0 , 1 month and 3 months. Polymorphic form has not changed.

The obtained film-coated tablets were subjected to the dissolution test in the phosphate buffer of pH 6.8.

The obtained film-coated tablets were further subjected to the disintegration test according to Ph. Eur. 10.0, 2.9.1 *"Disintegration of tablets and capsules",* Test A, without disc, in purified water, 750 ml, with the distinction that the temperature was measured at 25 degC. Tablets according to Examples 1 and 2 disintegrate in 12 minutes comparing to reference product Lixiana, which disintegrate only in 40 minutes.

### Examples 3 and 4:

In the same way as for Example 1 above Examples 3 and 4 were prepared, having the following compositions:

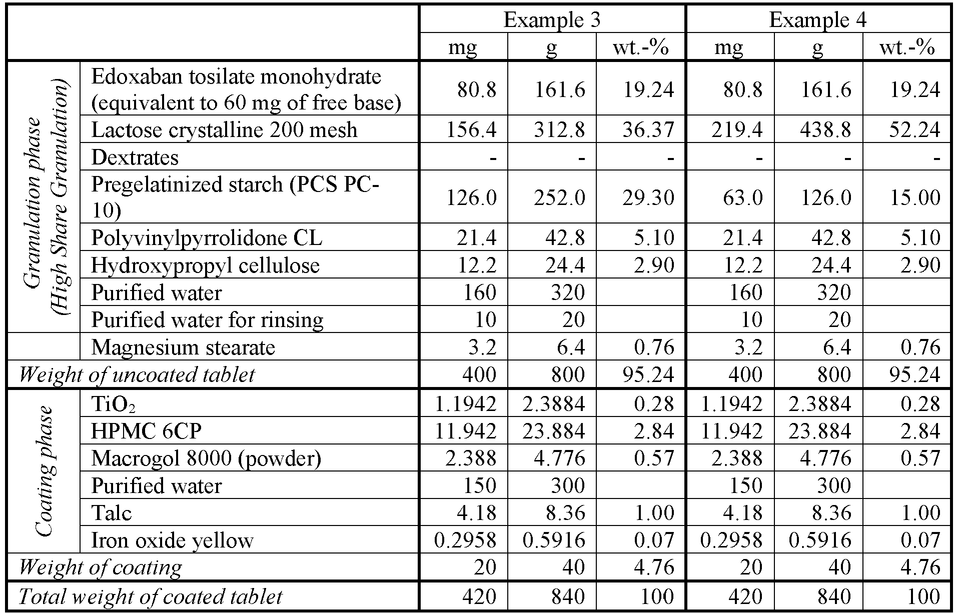

### Examples 5, 6 and 7:

In the same way as for Examples 1, 3 and 4 above Examples 5, 6 and 7 were prepared, with the exemption that all ingredients (including hydroxypropyl cellulose) of the granulation phase as shown in the above tables for Examples 1, 3 and 4, except for magnesium stearate, were mixed for 2 minutes. Then, the mixture was granulated for approximately 6 minutes by use of a high shear mixer while spraying the total quantity of purified water thereon, such that the loss on drying of the wet granules was 30 ± 2 percent, measured after heating a 2 g sample of wet granules at 85°C through absorption of IR radiation from a halogen radiator.

### Examples 8 and 9:

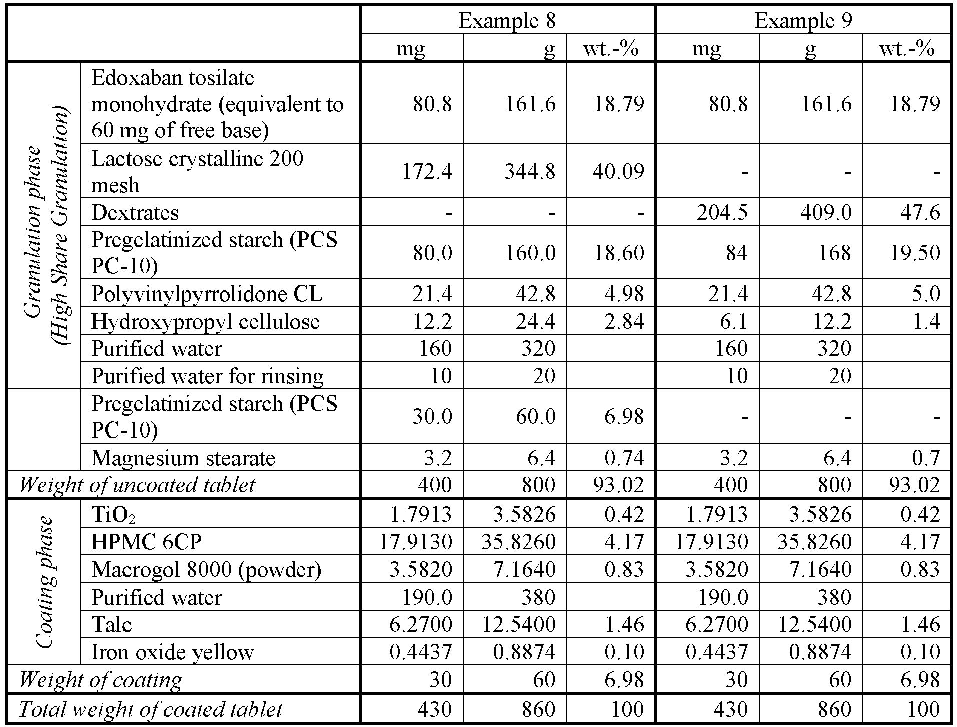

*Example 8* - Ingredients of the granulation phase as shown in the above table for Example 8, except for hydroxypropyl cellulose, magnesium stearate and part of pregelatinized starch, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 30 ± 2 percent by weight for, measured after heating a 2 g sample of wet granules at 85°C through absorption of IR radiation from a halogen radiator.

Then, the wet granules were dried at inlet air temperature of 65°C until the granulate temperature reached 41°C, such that the loss on drying of the dried granules was 1 to 3 percent, measured after heating a 2 g sample of dried granules at 85°C through absorption of IR radiation from a halogen radiator.

The thus produced wet granules were mixed with the second part of pregelatinized starch and additionally with magnesium stearate, and compressed into tablets (round, 11 mm in diameter, compression pressure: approximately 11 kN) by use of a tableting machine (Kilian Pressima). In this way, uncoated tablets having average hardness of 111 N were obtained.

*Example* 9 - Ingredients of the granulation phase as shown in the above table for Example 9, except for hydroxypropyl cellulose and magnesium stearate, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 33 ± 2 for, measured after heating a 2 g sample of wet granules at 85°C through absorption of IR radiation from a halogen radiator.

Then, the wet granules were dried at inlet air temperature of 65°C until the granulate temperature reached 41°C, such that the loss on drying of the dried granules was 6 to 8 percent by weight, measured after heating a 2 g sample of dried granules at 85°C through absorption of IR radiation from a halogen radiator.

The thus produced wet granules were mixed with magnesium stearate and compressed into tablets (round, 11 mm in diameter, compression pressure: approximately 11 kN) by use of a tableting machine (Kilian Pressima). In this way, uncoated tablets having average hardness of 111 N were obtained.

### Examples 10 and 11:

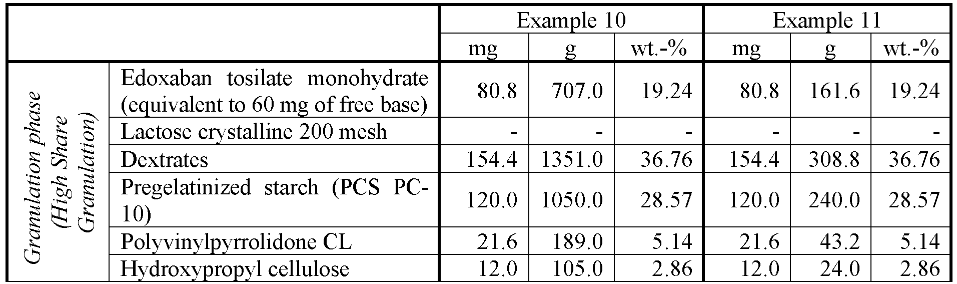

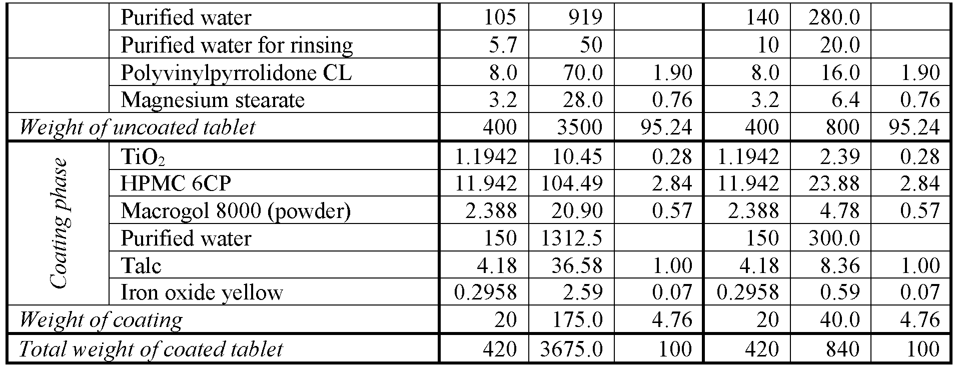

*Example 10* - Ingredients of the granulation phase as shown in the above table for Example 10, except for hydroxypropyl cellulose, magnesium stearate and part of Polyvinylpyrrolidone CL, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 27±2 percent by weight for, measured after heating a 2 g sample of wet granules at 85°C through absorption of IR radiation from a halogen radiator.

Then, the wet granules were dried at inlet air temperature of 65°C until the granulate temperature reached 34°C, such that the loss on drying of the dried granules was 5 to 7 percent, measured after heating a 2 g sample of dried granules at 85°C through absorption of IR radiation from a halogen radiator.

The thus produced wet granules were mixed with the second part of Polyvinylpyrrolidone CL and additionally with magnesium stearate, and compressed into tablets (round, 10 mm in diameter, compression pressure: approximately 10 kN) by use of a tableting machine (Kilian KTP). In this way, uncoated tablets having average hardness of 114 N were obtained.

Furthermore, ingredients of the coating phase, as shown in the above table were mixed with water to obtain an aqueous suspension. Film-coated tablets were produced by use of an aqueous suspension and a pan coater (Ima Perfima Lab).

*Example 11* - Ingredients of the granulation phase as shown in the above table for Example 11, except for hydroxypropyl cellulose, magnesium stearate and part of Polyvinylpyrrolidone CL, were mixed for 2 minutes. Then, the mixture was granulated for approximately 8 minutes by use of a high shear mixer while spraying of aqueous hydroxypropyl cellulose solution thereon. Additionally, purified water for rinsing the spraying system was sprayed thereon, such that the loss on drying of the wet granules was 30±2 percent by weight for, measured after heating a 2 g sample of wet granules at 85°C through absorption of IR radiation from a halogen radiator.

Then, the wet granules were dried at inlet air temperature of 65°C until the granulate temperature reached 34°C, such that the loss on drying of the dried granules was 5 to 7 percent, measured after heating a 2 g sample of dried granules at 85°C through absorption of IR radiation from a halogen radiator.

The thus produced wet granules were mixed with the second part of Polyvinylpyrrolidone CL and additionally with magnesium stearate, and compressed into tablets (round, 10 mm in diameter, compression pressure: approximately 11 kN) by use of a tableting machine (Kilian Pressima). In this way, uncoated tablets having average hardness of 116 N were obtained.

Furthermore, ingredients of the coating phase, as shown in the above table were mixed with water to obtain an aqueous suspension. Film-coated tablets were produced by use of an aqueous suspension and a pan coater (Glatt GC II).

### Examples 12 and 13:

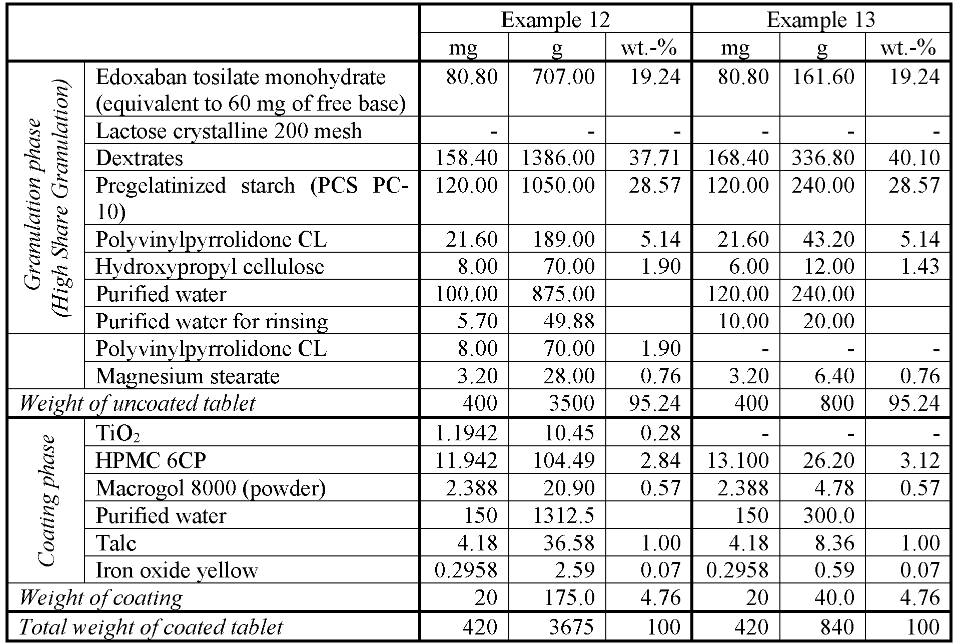

*Example 12* - Ingredients of the granulation phase as shown in the above table for Example 12, except for hydroxypropyl cellulose, magnesium stearate and part of Polyvinylpyrrolidone CL, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 27±2 percent by weight for, measured after heating a 2 g sample of wet granules at 85°C through absorption of IR radiation from a halogen radiator.

Then, the wet granules were dried at inlet air temperature of 65°C until the granulate temperature reached 36°C, such that the loss on drying of the dried granules was 5 to 7 percent, measured after heating a 2 g sample of dried granules at 85°C through absorption of IR radiation from a halogen radiator.

The thus produced wet granules were mixed with the second part of Polyvinylpyrrolidone CL and additionally with magnesium stearate, and compressed into tablets (round, 10 mm in diameter, compression pressure: approximately 11 kN) by use of a tableting machine (Kilian KTP). In this way, uncoated tablets having average hardness of 109 N were obtained.

Furthermore, ingredients of the coating phase, as shown in the above table were mixed with water to obtain an aqueous suspension. Film-coated tablets were produced by use of an aqueous suspension and a pan coater (Ima Perfima Lab).

*Example 13* - Ingredients of the granulation phase as shown in the above table for Examples 13, except for hydroxypropyl cellulose and magnesium stearate, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 29±2 percent by weight for, measured after heating a 2 g sample of wet granules at 85°C through absorption of IR radiation from a halogen radiator.

Then, the wet granules were dried at inlet air temperature of 65°C until the granulate temperature reached 35°C, such that the loss on drying of the dried granules was 5 to 7 percent, measured after heating a 2 g sample of dried granules at 85°C through absorption of IR radiation from a halogen radiator.

The thus produced wet granules were mixed with magnesium stearate and compressed into tablets (round, 10 mm in diameter, compression pressure: approximately 12 kN) by use of a tableting machine (Kilian Pressima). In this way, uncoated tablets having average hardness of 110 N were obtained.

Furthermore, ingredients of the coating phase, as shown in the above table were mixed with water to obtain an aqueous suspension. Film-coated tablets were produced by use of an aqueous suspension and a pan coater (Glatt GC II).

### Example 14:

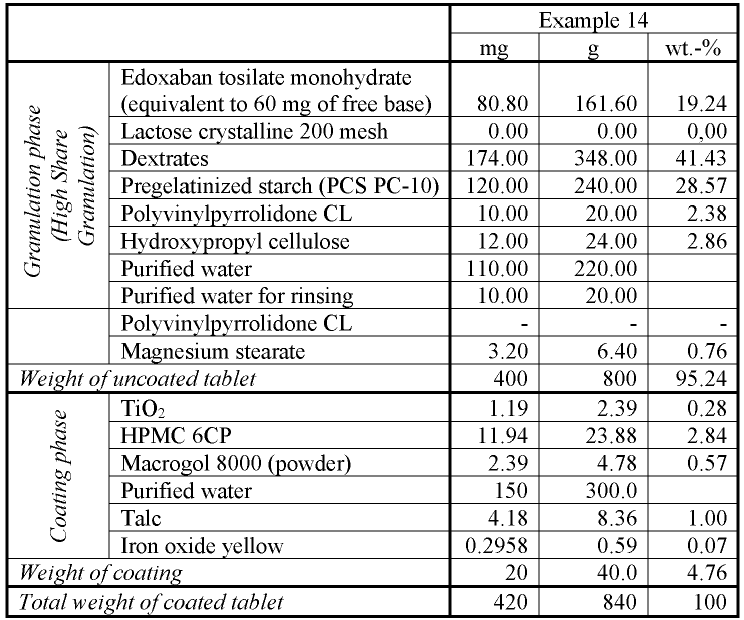

*Example 14* - Ingredients of the granulation phase as shown in the above table for Example 14, except for hydroxypropyl cellulose and magnesium stearate, were mixed for 2 minutes. Then, the mixture was granulated for approximately 9 minutes by use of a high shear mixer while spraying of aqueous hydroxypropyl cellulose solution thereon. Additionally, purified water for rinsing the spraying system was sprayed thereon, such that the loss on drying of the wet granules was 29±2 percent by weight for, measured after heating a 2 g sample of wet granules at 85°C through absorption of IR radiation from a halogen radiator.

Then, the wet granules were dried at inlet air temperature of 65°C until the granulate temperature reached 35°C, such that the loss on drying of the dried granules was 5 to 7 percent, measured after heating a 2 g sample of dried granules at 85°C through absorption of IR radiation from a halogen radiator.

The thus produced wet granules were mixed with magnesium stearate and compressed into tablets (round, 10 mm in diameter, compression pressure: approximately 11 kN) by use of a tableting machine (Kilian Pressima). In this way, uncoated tablets having average hardness of 105 N were obtained.

Furthermore, ingredients of the coating phase, as shown in the above table were mixed with water to obtain an aqueous suspension. Film-coated tablets were produced by use of an aqueous suspension and a pan coater (Glatt GC II).

## Claims

1. A tablet comprising a core and optionally a coating encapsulating said core, wherein the core comprises or essentially consists of
- Edoxaban, a pharmaceutically acceptable salt and/or solvate thereof;
- one or more saccharides comprising or essentially consisting of a mixture of glucose and maltose, optionally together with one or more oligosaccharides, being dextrates;
- optionally, one or more binders;
- optionally, one or more disintegrants; and
- optionally, one or more lubricants;
wherein the core does not comprise sugar alcohol.

2. The tablet according to claim 1, wherein the one or more saccharides comprise or essentially consist of a mixture of glucose and maltose together with one or more oligosaccharides; in addition to the glucose, maltose and one or more oligosaccharides, the core preferably contains neither any other monosaccharide nor any other disaccharide.

3. The tablet according to any of the preceding claims, wherein the weight content of the one or more saccharides, which is a mixture of glucose and maltose, optionally together with one or more oligosaccharides, is at least 10 wt.-%; preferably at least 15 wt.-%, more preferably at least 20 wt.-%, still more preferably at least 25 wt.-%, yet more preferably at least 30 wt.-%, even more preferably at least 35 wt.-%, most preferably at least 40 wt.-%, and in particular at least 45 wt.-%; in each case relative to the total weight of the one or more saccharides and relative to the total weight of the tablet.

4. The tablet according to any of the preceding claims, wherein the weight content of the one or more saccharides, which is a mixture of glucose and maltose, optionally together with one or more oligosaccharides, is at most 90 wt.-%; preferably at most 85 wt.-%, more preferably at most 80 wt.-%, still more preferably at most 75 wt.-%, yet more preferably at most 70 wt.-%, even more preferably at most 65 wt.-%, most preferably at most 60 wt.-%, and in particular at most 55 wt.-%; in each case relative to the total weight of the one or more saccharides and relative to the total weight of the tablet.

5. The tablet according to any of the preceding claims, wherein the weight content of the one or more saccharides, which is a mixture of glucose and maltose, optionally together with one or more oligosaccharides, is within the range of from 0.1 to 80 wt.-%, preferably 0.1 to 70 wt.-%, more preferably 0.1 to 60 wt.-%, still more preferably 0.1 to 50 wt.-%, yet more preferably 0.1 to 40 wt.-%, most preferably 0.1 to 30 wt.-%; in each case relative to the total weight of the one or more saccharides and relative to the total weight of the tablet.

6. The tablet according to any of the preceding claims, wherein the weight content of the one or more saccharides, which is a mixture of glucose and maltose, optionally together with one or more oligosaccharides, is within the range of from 1.0 to 60 wt.-%, preferably 1.0 to 50 wt.-%; in each case relative to the total weight of the one or more saccharides and relative to the total weight of the tablet.

7. The tablet according to any of the preceding claims, wherein the weight content of the one or more saccharides, which is a mixture of glucose and maltose, optionally together with one or more oligosaccharides, is within the range of 48±16 wt.-%; preferably 48±14 wt.-%, more preferably 48±12 wt.-%, still more preferably 48±10 wt.-%, yet more preferably 48±8.0 wt.-%, even more preferably 48±6.0 wt.-%, most preferably 48±4.0 wt.-%, and in particular 48±2.0 wt.-%; in each case relative to the total weight of the one or more saccharides and relative to the total weight of the tablet.

8. The tablet according to any of the preceding claims, wherein the Edoxaban, pharmaceutically acceptable salt and/or solvate thereof is Edoxaban tosilate.

9. The tablet according to any of the preceding claims, wherein the weight content of the Edoxaban, pharmaceutically acceptable salt and/or solvate thereof is at least 4.0 wt.-%; preferably at least 6.0 wt.-%, more preferably at least 8.0 wt.-%, still more preferably at least 10 wt.-%, yet more preferably at least 12 wt.-%, even more preferably at least 14 wt.-%, most preferably at least 16 wt.-%, and in particular at least 18 wt.-%; in each case relative to the given weight of the Edoxaban, pharmaceutically acceptable salt and/or solvate thereof and relative to the total weight of the tablet.

10. The tablet according to any of the preceding claims, wherein the one or more binders comprise or consist of
(i) cellulose or nonionic cellulose ethers; such as crystalline cellulose, methyl cellulose, ethyl cellulose, hydroxyethyl cellulose, hydroxypropyl cellulose, hydroxyethylmethyl cellulose, hydroxypropylmethyl cellulose (e.g. 2208, 2906, or 2910);
(ii) cellulose esters; such as cellulose acetate phthalate, or hydroxypropylmethyl cellulose phthalate;
(iii) starch and starch derivatives except pregelatinized starch and sodium starch glycolate; such as starch, flour, wheat starch, rice powder, rice starch, cornstarch, potato starch, or hydroxypropyl starch;
(iv) gums; such as alginic acid, sodium alginate, propylene glycol alginate ester, agar, gum arabic, arabinogalactan, tragacanth gum, locust bean gum, acacia gum, xanthan gum, pectin, gelatin, purified gelatin, or hydrolyzed gelatin powder;
(v) glycerin and polyethylene glycol; such as concentrate glycerin, or Macrogol (e.g. 4000, 6000, or 20000);
(vi) silicious materials; such as hydrous silicon dioxide, highly disperse silicon dioxide, light anhydrous silicic acid, or synthetic aluminum silicate;
(vii) vinyl polymers except crospovidone; such as carboxyl vinyl polymer, polyvinyl acetate, povidone, copovidone, copolydone, high molecular weight polyvinylpyrrolidone, polyvinyl alcohol (completely saponified product), polyvinyl alcohol (partially saponified product), vinylpyrrolidone-vinyl acetate copolymer, or sodium polyacrylate;
(viii) sodium polyphosphate; or mixtures thereof.

11. The tablet according to any of the preceding claims, wherein the one or more disintegrants comprise or consist of pregelatinized starch, crospovidone, sodium starch glycolate, carmellose, carmellose sodium, croscarmellose, croscarmellose sodium, or mixtures thereof.

12. The tablet according to any of the preceding claims, wherein the one or more lubricants comprise or consist of magnesium stearate, calcium stearate, stearic acid, stearyl alcohol, polyoxyl stearate, calcium carbonate, magnesium oxide, talc, bees wax, carnauba wax, aluminum hydroxide, fumaric acid, or mixtures thereof; preferably magnesium stearate.

13. The tablet according to any of the preceding claims, for use in the prevention and/or treatment of disease, disorder or condition that can be prevented and/or treated by inhibition of activated blood coagulation factor X (FXa).

14. A process for the preparation of a tablet according to any of the preceding claims, comprising wet granulation.

15. A tablet obtainable by the process according to claim 14.

## Patentansprüche

1. Eine Tablette, umfassend einen Kern und optional eine den Kern umhüllende Beschichtung, wobei der Kern umfasst oder im Wesentlichen besteht aus
- Edoxaban, dessen pharmazeutisch verträgliches Salz und/oder Solvat;
- einem oder mehreren Sacchariden, die eine Mischung aus Glucose und Maltose umfassen oder im Wesentlichen daraus bestehen, gegebenenfalls zusammen mit einem oder mehreren Oligosacchariden, die Dextrate sind;
- optional ein oder mehrere Bindemittel;
- optional ein oder mehrere Sprengmittel; und
- optional ein oder mehrere Gleitmittel;
wobei der Kern keinen Zuckeralkohol enthält.

2. Die Tablette nach Anspruch 1, wobei das eine oder die mehreren Saccharide eine Mischung aus Glucose und Maltose zusammen mit einem oder mehreren Oligosacchariden umfassen oder im Wesentlichen daraus bestehen; zusätzlich zu Glucose, Maltose und einem oder mehreren Oligosacchariden enthält der Kern bevorzugt weder ein anderes Monosaccharid noch ein anderes Disaccharid.

3. Die Tablette nach einem der vorstehenden Ansprüche, wobei der Gewichtsanteil des einen oder der mehreren Saccharide, die eine Mischung aus Glucose und Maltose, gegebenenfalls zusammen mit einem oder mehreren Oligosacchariden, sind, mindestens 10 Gew.-% beträgt; bevorzugt mindestens 15 Gew.-%, besonders bevorzugt mindestens 20 Gew.-%, noch mehr bevorzugt mindestens 25 Gew.-%, noch mehr bevorzugt mindestens 30 Gew.-%, noch mehr bevorzugt mindestens 35 Gew.-%, am meisten bevorzugt mindestens 40 Gew.-% und insbesondere mindestens 45 Gew.-%; jeweils bezogen auf das Gesamtgewicht des einen oder der mehreren Saccharide und bezogen auf das Gesamtgewicht der Tablette.

4. Die Tablette nach einem der vorstehenden Ansprüche, wobei der Gewichtsanteil des einen oder der mehreren Saccharide, die eine Mischung aus Glucose und Maltose, gegebenenfalls zusammen mit einem oder mehreren Oligosacchariden, sind, höchstens 90 Gew.-% beträgt; bevorzugt höchstens 85 Gew.-%, besonders bevorzugt höchstens 80 Gew.-%, noch mehr bevorzugt höchstens 75 Gew.-%, noch mehr bevorzugt höchstens 70 Gew.-%, noch mehr bevorzugt höchstens 65 Gew.-%, am meisten bevorzugt höchstens 60 Gew.-% und insbesondere höchstens 55 Gew.-%; jeweils bezogen auf das Gesamtgewicht des einen oder der mehreren Saccharide und bezogen auf das Gesamtgewicht der Tablette.

5. Die Tablette nach einem der vorstehenden Ansprüche, wobei der Gewichtsanteil des einen oder der mehreren Saccharide, die eine Mischung aus Glucose und Maltose, gegebenenfalls zusammen mit einem oder mehreren Oligosacchariden, sind, im Bereich von 0,1 bis 80 Gew.-%, bevorzugt 0,1 bis 70 Gew.-%, besonders bevorzugt 0,1 bis 60 Gew.-%, noch mehr bevorzugt 0,1 bis 50 Gew.-%, noch mehr bevorzugt 0,1 bis 40 Gew.-%, am meisten bevorzugt 0,1 bis 30 Gew.-% liegt; jeweils bezogen auf das Gesamtgewicht des einen oder der mehreren Saccharide und bezogen auf das Gesamtgewicht der Tablette.

6. Die Tablette nach einem der vorstehenden Ansprüche, wobei der Gewichtsgehalt des einen oder der mehreren Saccharide, die eine Mischung aus Glucose und Maltose, gegebenenfalls zusammen mit einem oder mehreren Oligosacchariden, sind, im Bereich von 1,0 bis 60 Gew.-%, bevorzugt 1,0 bis 50 Gew.-% liegt, jeweils bezogen auf das Gesamtgewicht des einen oder der mehreren Saccharide und bezogen auf das Gesamtgewicht der Tablette.

7. Die Tablette nach einem der vorstehenden Ansprüche, wobei der Gewichtsanteil des einen oder der mehreren Saccharide, die eine Mischung aus Glucose und Maltose, gegebenenfalls zusammen mit einem oder mehreren Oligosacchariden, sind, im Bereich von 48 ± 16 Gew.-% liegt; bevorzugt 48 ± 14 Gew.-%, besonders bevorzugt 48 ± 12 Gew.-%, noch mehr bevorzugt 48 ± 10 Gew.-%, noch mehr bevorzugt 48 ± 8,0 Gew.-%, noch mehr bevorzugt 48 ± 6,0 Gew.-%, am meisten bevorzugt 48 ± 4,0 Gew.-% und insbesondere 48 ± 2,0 Gew.-% liegt, jeweils bezogen auf das Gesamtgewicht des einen oder der mehreren Saccharide und bezogen auf das Gesamtgewicht der Tablette.

8. Die Tablette nach einem der vorstehenden Ansprüche, wobei das Edoxaban, dessen pharmazeutisch verträgliches Salz und/oder Solvat Edoxaban-Tosilat ist.

9. Die Tablette gemäß einem der vorstehenden Ansprüche, wobei der Gewichtsanteil des Edoxabans, dessen pharmazeutisch verträglichen Salzes und/oder Solvats mindestens 4,0 Gew.-% beträgt; bevorzugt mindestens 6,0 Gew.-%, besonders bevorzugt mindestens 8,0 Gew.-%, noch mehr bevorzugt mindestens 10 Gew.-%, noch mehr bevorzugt mindestens 12 Gew.-%, noch mehr bevorzugt mindestens 14 Gew.-%, am meisten bevorzugt mindestens 16 Gew.-% und insbesondere mindestens 18 Gew.-%; jeweils bezogen auf das angegebene Gewicht des Edoxaban, dessen pharmazeutisch verträglichen Salzes und/oder Solvats und bezogen auf das Gesamtgewicht der Tablette.

10. Die Tablette nach einem der vorstehenden Ansprüche, wobei das eine oder die mehreren Bindemittel umfassen oder bestehen aus
(i) Cellulose oder nichtionische Celluloseether; wie kristalline Cellulose, Methylcellulose, Ethylcellulose, Hydroxyethylcellulose, Hydroxypropylcellulose, Hydroxyethylmethylcellulose, Hydroxypropylmethylcellulose (z. B. 2208, 2906 oder 2910);
(ii) Celluloseester, wie Celluloseacetatphthalat oder HydroxypropylmethylcellulosePhthalat;
(iii) Stärke und Stärkederivate, ausgenommen vorgelatinierte Stärke und Natriumstärkeglycolat; wie Stärke, Mehl, Weizenstärke, Reispulver, Reisstärke, Maisstärke, Kartoffelstärke oder Hydroxypropylstärke;
(iv) Gummi, wie Alginsäure, Natriumalginat, Propylenglykolalginatester, Agar, Gummi arabicum, Arabinogalactan, Tragantgummi, Johannisbrotkernmehl, Akaziengummi, Xanthangummi, Pektin, Gelatine, gereinigte Gelatine oder hydrolysiertes Gelatinepulver;
(v) Glycerin und Polyethylenglykol, wie konzentriertes Glycerin oder Macrogol (z. B. 4000, 6000 oder 20000);
(vi) kieselsäurehaltige Stoffe, wie wasserhaltiges Siliciumdioxid, hochdisperses Siliciumdioxid, leichte wasserfreie Kieselsäure oder synthetisches Aluminiumsilikat;
(vii) Vinylpolymere außer Crospovidon, wie Carboxylvinyldimethacrylat, Polyvinylacetat, Povidon, Copovidon, Copolydon, hochmolekulares Polyvinylpyrrolidon, Polyvinylalkohol (vollständig verseiftes Produkt), Polyvinylalkohol (teilweise verseiftes Produkt), Vinylpyrrolidon-Vinylacetat-Copolymer oder Natriumpolyacrylat;
(viii) Natriumpolyphosphat; oder Mischungen davon.

11. Die Tablette nach einem der vorstehenden Ansprüche, wobei das eine oder die mehreren Sprengmittel vorgelatinierte Stärke, Crospovidon, Natriumstärkeglycolat, Carmellose, Carmellosenatrium, Croscarmellose, Croscarmellosenatrium oder Mischungen davon umfassen oder daraus bestehen.

12. Die Tablette gemäß einem der vorstehenden Ansprüche, wobei das eine oder die mehreren Gleitmittel Magnesiumstearat, Calciumstearat, Stearinsäure, Stearylalkohol, Polyoxylstearat, Calciumcarbonat, Magnesiumoxid, Talk, Bienenwachs, Carnaubawachs, Aluminiumhydroxid, Fumarsäure oder Mischungen davon umfassen oder daraus bestehen; bevorzugt Magnesiumstearat.

13. Die Tablette nach einem der vorstehenden Ansprüche, zur Verwendung bei der Vorbeugung und/oder Behandlung von Krankheiten, Störungen oder Zuständen, die durch Hemmung des aktivierten Blutgerinnungsfaktors X (FXa) verhindert und/oder behandelt werden können.

14. Ein Verfahren zur Herstellung einer Tablette gemäß einem der vorstehenden Ansprüche, umfassend eine Nassgranulierung.

15. Eine Tablette, erhältlich durch das Verfahren gemäß Anspruch 14.

## Revendications

1. Comprimé comprenant un noyau et éventuellement un enrobage encapsulant ledit noyau, dans lequel le noyau comprend ou est essentiellement constitué de
- Édoxaban, un sel et/ou un solvate pharmaceutiquement acceptable de celui-ci ;
- un ou plusieurs saccharides comprenant ou constitués essentiellement d'un mélange de glucose et de maltose, éventuellement avec un ou plusieurs oligosaccharides, étant des dextrates ;
- éventuellement, un ou plusieurs liants ;
- éventuellement, un ou plusieurs désintégrants ; et
- éventuellement, un ou plusieurs lubrifiants ;
dans lequel le noyau ne comprend pas d'alcool de sucre.

2. Comprimé selon la revendication 1, dans lequel les un ou plusieurs saccharides comprennent ou sont essentiellement constitués d'un mélange de glucose et de maltose avec un ou plusieurs oligosaccharides ; en plus du glucose, du maltose et des un ou plusieurs oligosaccharides, le noyau ne contient de préférence aucun autre monosaccharide ni aucun autre disaccharide.

3. Comprimé selon l'une quelconque des revendications précédentes, dans lequel la teneur en poids des un ou plusieurs saccharides, qui est un mélange de glucose et de maltose, éventuellement avec un ou plusieurs oligosaccharides, est d'au moins 10 % en poids ; de préférence d'au moins 15 % en poids, de manière davantage préférée d'au moins 20 % en poids, de manière encore davantage préférée d'au moins 25 % en poids, de manière davantage préférée encore d'au moins 30 % en poids, voire de manière davantage préférée d'au moins 35 % en poids, de manière préférée entre toutes d'au moins 40 % en poids, et en particulier d'au moins 45 % en poids ; dans chaque cas par rapport au poids total des un ou plusieurs saccharides et par rapport au poids total du comprimé.

4. Comprimé selon l'une quelconque des revendications précédentes, dans lequel la teneur en poids des un ou plusieurs saccharides, qui est un mélange de glucose et de maltose, éventuellement avec un ou plusieurs oligosaccharides, est d'au plus 90 % en poids ; de préférence d'au plus 85 % en poids, de manière davantage préférée d'au plus 80 % en poids, de manière encore davantage préférée d'au plus 75 % en poids, de manière davantage préférée encore d'au plus 70 % en poids, voire de manière davantage préférée d'au plus 65 % en poids, de manière préférée entre toutes d'au plus 60 % en poids, et en particulier d'au plus 55 % en poids ; dans chaque cas par rapport au poids total des un ou plusieurs saccharides et par rapport au poids total du comprimé.

5. Comprimé selon l'une quelconque des revendications précédentes, dans lequel la teneur en poids des un ou plusieurs saccharides, qui est un mélange de glucose et de maltose, éventuellement avec un ou plusieurs oligosaccharides, est comprise dans la plage de 0,1 à 80 % en poids, de préférence de 0,1 à 70 % en poids, de manière davantage préférée de 0,1 à 60 % en poids, de manière encore davantage préférée de 0,1 à 50 % en poids, de manière davantage préférée encore de 0,1 à 40 % en poids, et de manière préférée entre toutes de 0,1 à 30 % en poids ; dans chaque cas par rapport au poids total des un ou plusieurs saccharides et par rapport au poids total du comprimé.

6. Comprimé selon l'une quelconque des revendications précédentes, dans lequel la teneur en poids des un ou plusieurs saccharides, qui est un mélange de glucose et de maltose, éventuellement avec un ou plusieurs oligosaccharides, est comprise dans la plage de 1,0 à 60 % en poids, de préférence de 1,0 à 50 % en poids ; dans chaque cas par rapport au poids total des un ou plusieurs saccharides et par rapport au poids total du comprimé.

7. Comprimé selon l'une quelconque des revendications précédentes, dans lequel la teneur en poids des un ou plusieurs saccharides, qui est un mélange de glucose et de maltose, éventuellement avec un ou plusieurs oligosaccharides, est comprise dans la plage de 48 ± 16 % en poids ; de préférence de 48 ± 14 % en poids, de manière davantage préférée de 48 ± 12 % en poids, de manière encore davantage préférée de 48 ± 10 % en poids, de manière davantage préférée encore de 48 ± 8,0 % en poids, voire de manière davantage préférée de 48 ± 6,0 % en poids, et de manière préférée entre toutes de 48 ± 4,0 % en poids, et en particulier de 48 ± 2,0 % en poids ; dans chaque cas par rapport au poids total des un ou plusieurs saccharides et par rapport au poids total du comprimé.

8. Comprimé selon l'une quelconque des revendications précédentes, dans lequel l'édoxaban, un sel et/ou un solvate pharmaceutiquement acceptable de celui-ci est le tosilate d'Edoxaban.

9. Comprimé selon l'une quelconque des revendications précédentes, dans lequel la teneur en poids de l'édoxaban, d'un sel et/ou d'un solvate pharmaceutiquement acceptable de celui-ci est d'au moins 4,0 % en poids ; de préférence d'au moins 6,0 % en poids, de manière davantage préférée d'au moins 8,0 % en poids, de manière encore davantage préférée d'au moins 10 % en poids, de manière davantage préférée encore d'au moins 12 % en poids, voire de manière davantage préférée d'au moins 14 % en poids, de manière préférée entre toutes d'au moins 16 % en poids, et en particulier d'au moins 18 % en poids ; dans chaque cas par rapport au poids donné de l'édoxaban, d'un sel et/ou d'un solvate pharmaceutiquement acceptable de celui-ci, et par rapport au poids total du comprimé.

10. Comprimé selon l'une quelconque des revendications précédentes, dans lequel les un ou plusieurs liants comprennent ou sont constitués de
(i) la cellulose ou les éthers de cellulose non ioniques ; tels que la cellulose cristalline, la méthylcellulose, l'éthylcellulose, l'hydroxyéthylcellulose, l'hydroxypropylcellulose, l'hydroxyéthylméthylcellulose, l'hydroxypropylméthylcellulose (par exemple 2208, 2906 ou 2910) ;
(ii) les esters de cellulose ; tels que l'acétate phtalate de cellulose ou le phtalate d'hydroxypropylméthylcellulose ;
(iii) l'amidon et les dérivés de l'amidon, à l'exception de l'amidon prégélatinisé et du glycolate d'amidon sodique ; tels que l'amidon, la farine, l'amidon de blé, la poudre de riz, l'amidon de riz, l'amidon de maïs, l'amidon de pomme de terre ou l'amidon hydroxypropylique ;
(iv) les gommes ; telles que l'acide alginique, l'alginate de sodium, l'ester d'alginate de propylène glycol, l'agar, la gomme arabique, l'arabinogalactane, la gomme adragante, la gomme de caroube, la gomme d'acacia, la gomme xanthane, la pectine, la gélatine, la gélatine purifiée ou la poudre de gélatine hydrolysée ;
(v) la glycérine et le polyéthylène glycol ; tels que la glycérine concentrée ou le macrogol (par exemple 4000, 6000 ou 20000) ;
(vi) les matériaux siliceux ; tels que le dioxyde de silicium hydraté, le dioxyde de silicium hautement dispersé, l'acide silicique anhydre léger ou le silicate d'aluminium synthétique ;
(vii) les polymères vinyliques, à l'exception de la crospovidone ; tels que le polymère carboxyvinylique, l'acétate de polyvinyle, la povidone, la copovidone, la copolydone, la polyvinylpyrrolidone de haut poids moléculaire, l'alcool polyvinylique (produit entièrement saponifié), l'alcool polyvinylique (produit partiellement saponifié), le copolymère vinylpyrrolidone-acétate de vinyle ou le polyacrylate de sodium ;
(viii) le polyphosphate de sodium ; ou leurs mélanges.

11. Comprimé selon l'une quelconque des revendications précédentes, dans lequel les un ou plusieurs désintégrants comprennent ou sont constitués d'amidon prégélatinisé, de crospovidone, de glycolate d'amidon sodique, de carmellose, de carmellose sodique, de croscarmellose, de croscarmellose sodique ou leurs mélanges.

12. Comprimé selon l'une quelconque des revendications précédentes, dans lequel les un ou plusieurs lubrifiants comprennent ou sont constitués de stéarate de magnésium, de stéarate de calcium, d'acide stéarique, d'alcool stéarylique, de stéarate de polyoxyle, de carbonate de calcium, d'oxyde de magnésium, de talc, de cire d'abeille, de cire de carnauba, d'hydroxyde d'aluminium, d'acide fumarique ou leurs mélanges ; de préférence du stéarate de magnésium.

13. Comprimé selon l'une quelconque des revendications précédentes, pour une utilisation dans la prévention et/ou le traitement d'une maladie, d'un trouble ou d'une affection qui peut être prévenu et/ou traité par l'inhibition du facteur de coagulation sanguine activé X (FXa).

14. Processus de préparation d'un comprimé selon l'une quelconque des revendications précédentes, comprenant une granulation humide.

15. Comprimé pouvant être obtenu par le processus selon la revendication 14.
